Europäisches Patentamt

European Patent Office

Office européen des brevets

(11) Publication number: **0 394 314 B2**

(12) **NEW EUROPEAN PATENT SPECIFICATION**

(45) Date of publication of the new patent specification: **06.12.95**

(51) Int. Cl.6: **A61K 9/50**

(21) Application number: **89900483.2**

(22) Date of filing: **18.11.88**

(86) International application number: **PCT/US88/03995**

(87) International publication number: **WO 89/04656 (01.06.89 89/12)**

(54) **MULTIPLE STEP ENTRAPMENT/LOADING PROCEDURE FOR PREPARING LIPOPHILIC DRUG-CONTAINING LIPOSOMES.**

(30) Priority: **18.11.87 US 122354**

(43) Date of publication of application: **31.10.90 Bulletin 90/44**

(45) Publication of the grant of the patent: **03.03.93 Bulletin 93/09**

(45) Mention of the opposition decision: **06.12.95 Bulletin 95/49**

(84) Designated Contracting States: **AT BE CH DE FR GB IT LI LU NL SE**

(56) References cited:
**EP-A- 0 290 296**
**WO-A-86/01102**
**WO-A-88/06442**
**US-A- 4 769 250**

**LIPOSOMES, 1983, Marcel Dekker Inc., N.Y., USA; D. W. DEAMER et al, "Liposome Preparation: Methods and Mechanisms", pp. 27-51**

(73) Proprietor: **VESTAR, INC.**
**650 Cliffside Drive**
**San Dimas, CA 91773 (US)**

(72) Inventor: **FORSSEN, Eric, Anton**
**5181 Princess Anne Road**
**La Canada, CA 91011 (US)**

(74) Representative: **Goldin, Douglas Michael et al**
**J.A. KEMP & CO.**
**14, South Square**
**Gray's Inn**
**London WC1R 5LX (GB)**

EP 0 394 314 B2

D. D. PERRIN et al, "Buffers for pH and Metal Ion Control", Research Organics Inc., Cleveland, Ohio, USA, pp. 157-161

LIPOSOMES - From Biophysics to Therapeutics, 1987, Marcel Dekker Inc., N.Y., USA; S. M. GRUNER; "Materials Properties of Liposomal Bilayers", pp. 1-38

BIOCHIMICA ET BIOPHYSICA ACTA 857, 1956, Elsevier, L. D. MAYER et al, "Uptake of Adriamycin into Large Unilamellar Vesicles in Response to a pH Gradient", pp. 123-126

BIOCHEMICAL CALCULATIONS, I. H. Segel, 2nd ed., 1976, John Wiley & Sons, pp. 403-406

**Description**

This invention relates to novel liposome-entrapped lipophilic drug compositions, to methods of preparing such compositions, and to methods of using such compositions as chemotherapeutic agents to deliver the encapsulated drugs within the bodies of mammals, including humans.

More particularly, this invention relates to a novel multistep entrapment/loading procedure for incorporating any cationic, lipophilic drug which can partition into a lipid bilayer, and especially an anthracycline antineoplastic agent used to treat tumorous or malignant conditions in mammals, including humans, within liposome micellar particles which have been formed as unilamellar or multilamellar lipid vesicles. This novel entrapment/loading procedure facilitates the passage of the drug from the lipid membrane into the internal aqueous space within the liposome, thereby increasing entrapment efficiency and thus the amount of the drug deliverable within the body, e.g., the amount of an antineoplastic agent deliverable to tumor tissues. In the case of antineoplastic agents, the other benefits which have come to be associated with liposome-entrapped anthracycline antineoplastic agent therapy are also provided: increased in vivo stability for the drug delivery system, increased specificity of delivery of the drug to the tumor tissue site(s), decreased cardiotoxicity, and the ability to produce, and thus administer, these drug delivery systems on a large scale.

Background of the Invention

The use of liposomes as biodegradable delivery systems for a variety of drugs, including their use to direct anthracycline antineoplastic agents to tumor tissue, increase the efficiency of the delivered anthracycline drug and reduce its cardiotoxicity, has been described in the scientific literature with increasing frequency for nearly twenty years. See for example U.S. Patents Nos. 3,993,754, issued November 23, 1976 to Rahman et al; 4,217,344, issued August 12, 1980 to Vanlerberghe et al; 4,235,871, issued November 25, 1980 to Papahadjopoulos et al; 4,241,046, issued December 23, 1980 to Papahadjopoulos et al; 4,263,428, issued April 21, 1981 to Apple et al; 4,310,505, issued January 12, 1982 to Baldeschwieler et al; 4,330,534, issued May 18, 1982 to Sakurai et al; 4,331,654, issued May 25, 1982 to Morris; 4,356,167, issued October 26, 1982 to Kelly; 4,411,894, issued October 25, 1983 to Schrank et al; 4,419,348, issued December 6, 1983 to Rahman et al; 4,427,649, issued January 24, 1984 to Dingle et al; 4,438,052, issued March 20, 1984 to Weder et al and 4,515,736, issued May 7, 1985 to Deamero Also, International Application No. PCT/US84/01431, published March 4, 1985 (International Publication NO. WO 85/00968) naming Mayhew et al as inventors; International Application No. PCT/US84/00855, published November 21, 1985 (International Publication No. WO 85/05030) naming Janoff et al as inventors; European Patent Application No. 0,004,467, published October 3, 1979 naming Apple et al as inventors; European Patent Application No. 0,036,676, published September 30, 1981 naming Hunt et al as inventors; European Patent Application No. 0,161,445, published November 21, 1985 naming Fukushima et al as inventors; European Patent Application No. 0,198,765, published October 22, 1986 naming Rahman as inventor, and British Patent Application No. GB 2,146,525A, published April 24, 1985 naming Margalit as inventor. Also, Ryman et al, "Possible Use of Liposomes in Drug Delivery", in "Optimization of Drug Delivery, Alfred Benzon Symposium 17" Ed. H. Bundgaard et al (Copenhagen: Munksgaard, 1982); Forssen et al, Proc, Natl, Acad, Sci. USA, 78, No. 3, 1873-1877 (1981); Gabizon *et al*, Cancer Research, 42, 4734-4739 (1982) and Forssen *et al*, Cancer Research, 43, 548-550 (1983).

One common drawback associated with prior art methods for preparing liposome drug delivery systems is the low levels of liposome drug entrapment (trapping efficiency) achievable by such methods. International Patent Applications Nos. PCT/US85/01501 and PCT/US85/01502, published February 27, 1986 (WO86/01102 and WO89/01103) naming Bally *et al* as inventors, disclose methods which are reported to provide increases in trapping efficiencies approaching 100% entrapment while, at the same time, increasing the rate at which the drug is loaded into the liposome carrier. These methods involve generating a transmembrane potential creating an ionic gradient for one or more charged species --Na + /K + , Ca$^{++}$ and H + are disclosed -- across the walls of the liposome. The concentration gradient, as the name implies, results from producing liposomes having different concentrations of charged species within (the internal phase) and outside of ("the external phase") the vesicles. And see Ostro, Ed. "Liposomes, from Biophysics to Therapeutics" (New York: Marcel Dekker, Inc., 1987), pp. 60-65.

Adding acids, bases or both to control pH while carrying out processes designed to produce small unilamellar liposomes from specified lipid mixtures is disclosed in U.S. Patent No. 4,619,794, issued October 28, 1986 to Hauser. The Hauser patent, however, does not disclose a method of loading drugs into preformed vesicles.

3

EP-A-290296, which forms part of the state of the art under Article 54(3) and (4) EPC for the present application, describes liposomal formulations made by a process in which the liposomes are formed in a first aqueous medium, preferably a buffer, then acidifying or alkalinizing the medium thereby establishing a pH gradient.

The resulting acidified or alkalinized liposomes are then mixed with an antineoplastic agent. The acid solutions disclosed are acetic acid buffer, oxalic acid buffer, succinic acid buffer and citric acid buffer. The pH of the acidic buffer is about 3.5 to about 4.5.

## Summary of the Invention

It has now been discovered that cationic, lipophilic drugs capable of partitioning into a lipid bilayer can be entrapped and loaded within typical liposome drug delivery systems rapidly and in high concentrations by a procedure which comprises, first of all, forming liposomes in aqueous medium containing an acid which is a monofunctional pyranocidal acid or citric acid, where the pH of the aqueous medium is from 2.0 to 2.5.

The drug being entrapped can be present while the liposomes are being formed or it can be added to the liposome-containing acidic aqueous medium (the external phase) subsequent to liposome formation, and in either case will become imbedded in but will not penetrate bilayer at this point in the process.

A base which will convert the acid molecules in the internal and external phases to the corresponding anions is then added. This base should be one whose cations cannot pass through the liposome vesicles' lipid bilayers, and the choice of a particular base will also be governed by the particular drug being entrapped. Certain bases have been found not to work, or to work less efficiently than others, in entrapping certain drugs, and particularly anthracycline antineoplastic agents.

The base cations charge neutralize the acid anions in the external aqueous phase. However, since the base cations are unable to pass through the lipid bilayer, acid anions contained in the internal aqueous phase within the vesicles can become charge neutralized only by combining with the cationic drug. Thus, the drug is induced to pass from the membrane bilayer into the internal aqueous phase.

It is therefore, an object of this invention to provide novel cationic, liposome-entrapped lipophilic drug compositions, such as liposome-entrapped anthracycline antineoplastic agent compositions.

A further object of this invention is to provide a novel, multistep entrapment/loading method of preparing liposome entrapped cationic, lipophilic drug compositions, such as liposome-entrapped anthracycline antineoplastic agent compositions, which facilitates the passage of the drug from the lipid membrane into the internal aqueous space within the liposome, thereby increasing entrapment efficiency and thus the amount of drug deliverable to the appropriate site(s) within the body.

These and other objects, as well as the nature, scope and utilization of this invention, will become readily apparent to those skilled in the art from the following description and the appended claims.

## Detailed Description of the Invention

Included among the cationic, lipophilic drugs which can partition into a lipid bilayer and which are thus suitable for use in practicing this invention are:

| Drug Class | Examples |
|---|---|
| Local anesthetics | Dibucaine, tetracaine, procaine, chlorpromazine |
| Cholinergic agents | Pilocarpine, physostigmine, neostigmine |
| Antimalarial agents | Chloroquine, amodiaquine, chloroguanide, primaquine, mefloquine, quinine |
| Antiparkinson agents | Pridinol, prodipine, benztropine mesylate, trihexyphenidyl hydrochloride |
| Antagonists for adrenergic receptors | Propranolol, timolol, pindolol |
| Antiprotazoates | Pentamidine, quinacrine |
| Antihistamines | Benadryl, promethazine |
| Biogenic amines | Dopamine, seratonin, epinephrine |
| General analgesics | Codeine, meperidine, methadone, morphine |
| Anticholenergics | Atropine, decyclomine, methixene, propantheline |
| Antidepressants | Imipramine, amitriptyline, doxepin, desipramine |
| Antiarrhythmic agents | Quinidine, propranolol, lidocaine |
| Antiemetics | Chloropromazine, promethazine, perphenazine |

Ostro, op. cit., p. 64.

Cationic anthracycline compounds having antineoplastic activity against cancerous tissues or cells, including daunorubicin (also known as daunomycin), doxorubicin (also known as adriamycin), aclacinomycin A, vinblastine, vincristine, mitomycin C, and the like, are particularly preferred for incorporation within liposome micellar particles using the novel multistep entrapment/loading procedure of this invention. Structurally, these anthracycline compounds contain a hydrophobic tetracycline ring system coupled to an amino sugar through a glycoside linkage.

Biological lipids from which liposome bilayer membrane particles or vesicles useful in practicing this invention can be prepared are amphiphatic (hydrophobic and hydrophilic portion-containing) molecules which can spontaneously aggregate to form small spheres, ellipsoids or long cylinders, or bilayers having two or more parallel layers of amphiphatic molecules. In an aqueous (polar) medium, the polar heads of the amphiphatic molecules making up one layer orient outwardly to extend into the surrounding medium while the non-polar tail portions of these molecules likewise associate with each other. This provides a polar surface and a non-polar core in the wall of the vesicle. Such bilayered micelles usually take the shape of unilamellar (having one bilayer) or multilamellar (having a plurality of substantially concentric bilayers) spherical vesicles having an internal aqueous compartment.

Liposome bilayer membrane particles which have been found to be suitable in practicing this invention are small [e.g., from about 30 to about 150 nanometers (nm), and preferably from about 45 to about 60 nm, in diameter as determined, for example, using a light scattering particle sizer] neutral (uncharged or having balanced charges; i.e., zwitterions) unilamellar or multilamellar phospholipid vesicles or liposomes tailored to maximize entrapment/loading of a cationic, lipophilic drug by the method of this invention and to induce specificity and tissue/cell targeting, thereby maximizing uptake of the resulting liposome drug delivery system.

Liposomes suitable for use in practicing this invention can be prepared from carboxylic acid diesters of aliphatic triols and higher polyols, such as glycerol, sorbitol, mannitol, and the like, with glycerol being preferred, or of sphingosine or other amino alcohols containing long, unsaturated hydrocarbon chains, e.g., dialkyl amphiphiles such as sphingomyelin and the like, in which the ester moieties are derived from saturated or ethylenically unsaturated (from one to four, and preferably one or two unsaturated sites per chain) aliphatic monocarboxylic acids (long chain fatty acids) having from at least 14 to about 30 carbon atoms, and preferably from about 18 to about 24 carbon atoms, such as palmitic, stearic, 10-methylstearic, lignoceric, palmitoleic, oleic, linoleic, phytanic and arachidonic acids and the like, and in which one or more, preferably one, of the polyol's hydroxy groups is substituted with a phosphate ester group which will itself be substituted with lower aliphatic di-or higher functional compounds, generally lower aliphatic compounds having hydroxyl or amino (including substituted amino, e.g. lower alkyl substituted amino] groups, or both, such as ethanolamine, choline, serine, inositol, and the like.

Such liposome bilayer membrane particles include ones made from dipalmitoyl phosphatidylcholine, distearoyl phosphatidylcholine, dioleoyl phosphatidylethanolamine, distearoyl phosphatidylserine, dilinoleoyl phosphatidylinositol, distearoyl phosphatidylglycerol, and the like, or mixtures thereof. Liposome bilayer membrane particles made entirely from neutral phospholipids, such as distearoyl phosphatidylcholine, and preferably ones which have been further stabilized with cholesterol or like-acting substances, for example in a molar ratio of distearoyl phosphatidylcholine: cholesterol of about 2:1, respectively, have been found to be particularly suitable with regard to targeting efficiency when used to deliver anthracycline antineoplastic agents.

These liposomes are prepared by generally known techniques, such as the sonication method described in Mauk et al, Anal. Bioc, 94, 302-307 (1979) or by micro-emulsification using the procedure described in copending U.S. patent application Serial No. 696,727, filed January 31, 1985 in the name of R. Gamble and of common assignment with this application. Homogenization using a sonicator device will generally be carried out for from about 30 seconds to one minute per milliliter of suspension. Following homogenization, the suspension is centrifuged at from about 1,000 xg to about 20,000 xg, and preferably at about 5,000 xg, for from about 5 to 20 minutes, preferably about 10 minutes, at ambient temperature (usually about 22°C), and then pressed through a small pore diameter sterile filter, e.g., a 0.2-0.45 micron pore filter. These two steps (centrifugation and filtration) remove large particulate matter such as unsuspended lipids, large vesicles and other possible contaminating particles.

Permeability or leakage can be measured by separating the vesicles from any material which has leaked out, using methods such as gel permeation chromatography, dialysis, ultrafiltration or the like, and assaying in known manner for any leaked material. Permeabilities ranging from about one to about ten percent of the original entrapped material over a period of about 24 hours or longer, and preferably less than about one percent of the original entrapped material over a period of about 24 hours or longer, are acceptable when practicing this invention.

The acids that are used in practicing this invention are citric acid or a, monofunctional pyranosidyl acid such as glucuronic acid, gulonic acid, gluconic acid, galacturonic acid, glucoheptonic acid, actobionic acid.

The pyranosidyl acids have been found to be most effective for anthracycline antineoplastic agent loading by the method of this invention, inasmuch as doxorubicin, which has been found to be entrapped with more difficulty than daunorubicin by this method, is readily entrapped by lactobionic and galacturonic acids but not by acetic acid. Citric acid has also been found to entrap doxorubicin, but surprisingly the resulting loaded vesicles proved to be far more toxic than the free drug. In nearly all instances of vesicle-entrapped doxorubicin reported in the literature, the loaded vesicles are less toxic than the free drug.

The base which will convert the acid molecules in the internal and external aqueous phases to the corresponding anions should, as mentioned above, be one whose cations cannot pass through the vesicles lipid bilayers. Included among such bases are alkali and alkaline earth metal hydroxides, carbonates and like compounds, e.g., sodium, potassium, lithium, calcium and magnesium hydroxides and carbonates, and amines such as Nimethylglucamine, ethylene diamine and TRIS base [also known astromethamine, 2-amino-2-hydroxymethyl-1,3-propanediol and tris (hydroxymethyl)aminomethane], all of which have high solubility in water -- greater than 0.01 Molar -- and are of low solubility-- less than O.O1mMolar -- or insoluble in organic solvents such as ethanol, chloroform, diethyl ether and ethyl acetate.

Certain of the bases which meet this criterion of having cations which cannot pass through the vesicles lipid bilayers have been found to be more effective than others for anthracycline antineoplastic agent loading by the method of this invention. Carbonates, such as calcium carbonate, sodium carbonate, sodium bicarbonate, and the like have been found to work in all cases, including for the difficultly entrappable doxorubicin. Basic hydroxides, such as sodium, potassium and calcium hydroxide, and amines, including ethylene diamine, TRIS and N-methylglucamine all were not effective for loading doxorubicin into vesicles by the method of this invention.

In practicing the method of this invention the vesicles, e.g., as dry powders if stored in this form, are first dispersed in aqueous medium at a temperature of from about 40°C to about 80°, preferably from about 50°C to about 70°C, at a vesicle concentration of from about 5 mg/ml to about 100 mg/ml, and preferably at from about 20 mg/ml to about 40 mg/ml. Water alone, preferably although not necessarily deionized (small anions such as chloride, which can easily pass through the bilayer membrane, may interfere with the entrapment/loading procedure) may be used, or other low ionic media such as the aqueous sugar solutions disclosed in copending U.S. patent application Serial No. 787,535, filed October 15, 1985 in the name of Eric Forssen and of common assignment with this application (attorney's docket number 171/279).

As mentioned above cholesterol and like-acting substances, e.g., other sterols, zwitterionic or charged lipids, and the like, can be added, if desired, to the aqueous vesicle dispersion to further improve the stability of the subsequently-formed drug-loaded vesicles. Such substances ordinarily will be added in amounts ranging from about 0.1 to about 50 mol percent, and preferably from about 5 to about 33 mol percent, based on the total amount of the components of the bilayer membrane.

The chosen acid used when preparing the aqueous vesicle-containing medium will be present during hydration and homogenization (e.g., sonication) and before addition of the drug being loaded, and will be employed in concentrations ranging from about 10 mMolar (millimolar) to about 300 mMolar, and preferably from about 50 mMolar to about 200 mMolar, with the suspension being held at a temperature of from about 30°C to about 70°C, and preferably at from about 50°C to about 60°C, during this step.

The drug, e.g. an anthracyclic antineoplastic agent, will be added to the acidic aqueous vesicle-containing medium either as a dry powder or, preferably, as a concentrated solution in water, in amounts ranging from about 1mg/1OOmg lipid to about 1Omg/1OOmg lipid, and preferably from about 1mg/20mg lipid to about 1mg/30mg lipid, in order to insure as close to 100% entrapment as possible. The temperature at which the drug is added will generally be above the phase transition temperature of the vesicles, e.g., a temperature of from about 40°C to about 80°C, and preferably from about 50°C to about 65°C, to facilitate diffusion of the drug into the vesicles, and the drug-containing suspension will preferably be held at that temperature during subsequent base addition.

The selected base will be added to the drug-containing acidic vesicle suspension in amounts ranging from about one-sixth mol equivalent to about one mol equivalent, based on the net ionization of the acid and the final pH desired. In the case of anthracycline antineoplastic agent-containing suspensions, a pH ranging from about 4.5 to about 6.0 will usually be achieved. Base addition will ordinarily be carried out while continuing to heat the mixture, e.g., at a temperature of from about 40°C, over a period of from about one to about ten minutes, preferably while agitating the mixture to produce rapid and thorough mixing. Following base addition, heating will ordinarily be continued for another 5 to 20 minutes, preferably about 10 minutes.

The time periods within each of these method steps and the overall entrapment/loading procedure are carried out are, however, not critical. The overall method, including subsequent workup procedures to ready the drug delivery systems for parenteral administration, e.g., for human intravenous injection, or for storage until used will ordinarily take from about 30 to about 120 minutes.

Such workup procedures can include, first of all, removal of the acid or other anions from the aqueous environment external to the liposomes and its replacement with, e.g., an aqueous solution of a sugar, e.g., a solution containing from about 5% to about 20% by weight of a biologically acceptable mono-, di- or trisaccharide compatible with the drug-filled vesicles, such as lactose, dextrose, sucrose, trehalose, raffinose, maltose, or the like, or such a solution containing a non-saccharide polyol such as glycerol, inositol, sorbitol, mannitol, or the like, and also containing from about 0.1% to about 2% by weight of an excipient such as glycine, tromethamine, n-methylglucamine, or the like, which functions as a buffer. This removal of the acid or other anions from the external aqueous medium can be accomplished using gel permeation chromatography, ultrafiltration, vacuum dialysis, tangential flow filtration, hollow fiber filtration or like methods, with gel permeation chromatography being preferred for volumes of product of 100 ml or less and tangential flow filtration for volumes in excess of 100 ml.

Acid replacement is ordinarily followed by centrifugation and concentration, again using vacuum dialysis, ultrafiltration, tangential flow filtration or other methods designed to remove water and aqueous solutes while retaining the lipid vesicles and their contents.

In order that those skilled in the art can more fully understand this invention, the following examples are set forth. These examples are given solely for purposes of illustration, and should not be considered as expressing limitations unless so set forth in the appended claims. All parts and percentages are by weight, unless otherwise stated.

Example I

Entrapment of daunorubicin in distearoyl phosphatidylcholine-cholesterol vesicles using citric acid and sodium hydroxide.

A dry powder containing a homogenous dispersion of cholesterol in distearoyl phosphatidylcholine at a 1:2 molar ratio is prepared. This dispersion is then hydrated with an aqueous solution containing 41/2% lactose (equivalent to 125 mMolar) and 50 mM citric acid at a pH of about 2.0 to 2.5 (not adjusted). The concentration of lipid in the resulting suspension is 20 mg per ml. This mixture is heated to 65°C and an amount of daunorubicin is added as a concentrated solution in water sufficient to yield a final daunorubicin concentration of about 1.0 mg/ml. Next, while continuing to heat the mixture, an amount of sodium hydroxide equal to two and one half mole equivalents per mole of citric acid is added to the mixture over a period of about three minutes. The suspension is then vigorously agitated to produce rapid and thorough mixing.

Following the addition of sodium hydroxide, the mixture is incubated at the same temperature for about 10 minutes. At this stage in the procedure, the daunorubicin has transversed the bilayer membrane to the vesicle interior and formed a salt with citrate as the counterion. After the incubation period, the mixture is cooled to ambient temperature and centrifuged as described previously. Next, the exterior aqueous phase, containing citrate and sodium ions, along with any unentrapped daunorubicin if present, is exchanged for a solution of 9% lactose in water with 50 mMolar glycine by gel permeation chromatography.

Following this exchange procedure, the drug containing tangential flow filtration, which removes water and aqueous solutes while retaining the lipid vesicles and their contents. This concentrated product is then sterilized by filtration through 0.45 $\mu$m (micron) diameter pore filters. The finished lipid vesicle suspension containing entrapped daunorubicin is then ready for use or may be stored for four or more weeks frozen, or at from 4°C.

Example II

Entrapment of doxorubicin using distearoyl phosphatidylcholine, cholesterol, lactobionic acid and calcium carbonate.

A dry powder containing a homogenous dispersion of cholesterol in distearoyl phosphatidylcholine in a 1:2 mole ratio is prepared. This dispersion is then hydrated with an aqueous solution of 200 mMolar lactobionic acid in water (71.66 g/liter) with a pH of 2.0 to 2.5 (not adjusted}. The lipid concentration in the resulting suspension is about 20 mg/ml. This mixture is then heated to 70°C. The preparation is then homogenized, centrifuged and filtered as described in Example I hereinabove.

After the suspension of small, unilamellar vesicles in 200 mMolar lactobionic acid has been prepared, it is heated to 65°C and amount of doxorubicin is then added, as a concentrated solution in water, which will result in a final doxorubicin concentration of about 1.0 mg/ml. Next, while continuing to heat the mixture, calcium carbonate is added as a dry powder in an amount equivalent to about 20 grams per liter. Heating is continued for an additional 10 minutes, during which time the preparation is vigorously agitated. Next the mixture is centrifuged to remove excess calcium carbonate and other precipitated materials. The remaining processing steps are as described in Example I hereinabove.

A lipid vesicle suspension containing entrapped doxorubicin is obtained.

Example III

The procedure of Example II above is repeated in every essential detail except for the following:
- galacturonic acid is used in place of lactobionic acid;
- calcium carbonate is replaced by sodium carbonate.

A lipid vesicle suspension containing entrapped doxorubicin is again obtained.

Example IV

The procedure of Example II above is again repeated in every essential detail but one. Distearoyl phosphatyidylglycerol, cholesterol and distearoyl 15 phosphatidylcholine are included in the vesicle bilayer in a mol ratio of about 1.5:5:10, respectively.

A lipid vesicle suspension containing entrapped doxorubicin is again obtained.

Example V

Daunorubicin vesicles, prepared as described in Example I hereinabove, were stored frozen, refrigerated at 4°C, or at room temperature (22°C). At two and four weeks, the samples were assayed for: 1) chemical stability of the drug and lipid components, 2) retention of drug within the vesicles, and 3) biological activity as measured by antitumor activity (two weeks only). No significant degradation or loss of drug or of vesicle lipids could be detected out to four weeks. There also appeared to be no significant leakage except for possibly about a 5% leakage of drug from frozen and thawed vesicles. Biological activity was superior to freshly prepared free daunorubicin and slightly less than (4°C) freshly prepared vesicles.

Example VI

Antitumor activity of daunorubicin vesicles.

Daunorubicin vesicles prepared as described in Example I hereinabove have been tested against P-1798 lymphosarcoma and Ma16c mammary adenocarcinoma, both solid tumors in mice. The studies with the Ma16c tumor are still in progress; the P-1798 tumor studies have been completed. Treatments at all dose levels, ranging from 10 mg/kg to 50 mg/kg, have demonstrated that when formulated as described above daunorubicin vesicles have more antitumor activity and less toxicity, as determined by increased life span and reduced tumor size, than the parent drug.

Example VII

Biological activity of doxorubicin vesicles.

Doxorubicin vesicles prepared as described in Examples II and III have been tested for toxicity and antitumor activity. These studies are still continuing, however, some results and conclusions are now evident.

Treatment of CD2F1 mice has indicated that the maximum tolerated dose (MTD, non-lethal) of a single intravenous dose of the free drug, doxorubicin hydrochloride, is about 25 mg/kg. This compares to an MTD of about 40 mg/kg (normalized to the hydrochloride) for doxorubicin vesicles prepared as described in Example III. In contrast, the MTD for doxorubicin vesicles, when prepared as described in Example III with the exception that citric acid was substituted for lactobionic acid, was only about 10 mg/kg, less than the MTD for the free drug.

Doxorubicin vesicles prepared with lactobionic acid have demonstrated less toxicity as measured by suppression of white blood cells and by survival at high doses as compared to free drug.

White blood cell (w.b.c.) counts were performed on CD2F1 mice receiving doxorubicin doses of 40 mg/kg (for the equivalent hydrochloride). Mice receiving free drug experienced severe depression in w.b.c. counts: at four days post therapy, counts were only 8.5% of pretherapy values. In contrast, mice treated with doxorubicin vesicles prepared in accordance with this invention (Example III) at the same doxorubicin dosage experienced only moderate suppression in w.b.c. counts: at four days post therapy, counts were 75% of pretherapy values.

Doxorubicin vesicles can also be prepared with citric acid. However, intravenous tests in mice have demonstrated that while free drug doses of 20 mg/kg are tolerated, equivalent doses of doxorubicin citrate in vesicles are lethally toxic.

The above discussion of this invention is directed primarily to preferred embodiments and practices thereof. It will be readily apparent to those skilled in the art that further changes and modifications in the actual implementation of the concepts described herein can easily be made without departing from the invention as defined by the following claims.

## Claims

1. A method of preparing a phospholipid-entrapped cationic, lipophilic drug composition which comprises:
   a. forming liposomes in an aqueous medium containing an acid which is a monofunctional pyranosidyl acid or citric acid and where the pH of the aqueous medium is from 2.0 to 2.5, to give an acidic liposome-containing aqueous medium in which the acid is present in the internal and external liposome phases, said liposome being prepared from hydroxyamino(lower)aliphatic-substituted phosphatidyl carboxylic acid diesters of a tri- or higher functional aliphatic polyol in which the ester moieties are derived from a saturated or ethylenically unsaturated aliphatic monocarboxylic acid having at least 14 carbon atoms,
   b. adding to the thus-obtained acidic liposome-containing aqueous medium a cationic, lipophilic drug, and
   c. adding a base whose cations cannot pass through the liposomes' lipid bilayers to charge neutralize acid anions in the external aqueous phase, thereby inducing the cationic, lipophilic drug to pass into the liposomes' internal aqueous phase.

2. A method as recited in claim 1 in which the monofunctional pyranosidyl acid is glucaronic acid, gluconic acid, gulonic acid, galacturonic acid, glucoheptanoic acid or lactobionic acid.

3. A method as recited in any one of the preceding claims in which said acid is lactobionic acid.

4. A method as recited in any one of the preceding claims in which said base is an alkali or alkaline earth metal hydroxide or carbonate.

5. A method as recited in any one of the preceding claims in which, following the addition of the base, the acid and other anions are removed from the aqueous environment external to the liposomes and replaced with an aqueous sugar solution.

6. A method as recited in any one of the preceding claims in which said cationic, lipophilic drug is an anthracycline anti-neoplastic agent.

7. A method as recited in claim 1 in which said acid is citric acid, said base is sodium hydroxide, said diester is distearoyl phosphatidylcholine, said cationic, lipophilic drug is daunorubicin, and cholesterol is present in the liposome bilayers as a stabilizer.

8. A method as recited in claim 1 in which said acid is lactobionic acid, said base is sodium hydroxide, said diester is distearoyl phosphatidylcholine, said cationic, lipophilic drug is doxorubicin and cholesterol is present in the liposome bilayers as a stabilizer.

9. A phospholipid-entrapped cationic, lipophilic drug composition prepared by a method as described in any one of the preceding claims.

## Patentansprüche

1. Verfahren zur Herstellung einer in Phosholipid eingeschlossenen, kationischen, lipophilen Arzneimittelzusammensetzung, das folgende Verfahrensschritte umfasst:
   a) Erzeugen von Liposomen in einem wässrigen Medium, das eine monofunktionelle Pyranosidylsäure oder Zitronensäure enthält und wobei der pH des wässrigen Mediums von 2,0 bis 2,5 beträgt, um ein saures, liposomhaltiges wässriges Medium zu ergeben, bei dem die Säure in den internen und externen Liposomphasen vorliegt, wobei das Liposom aus Hydroxyaminoaliphat(niederer Kettenlänge)-substituierten Phosphatidylcarbonsäurediestern eines tri- oder höherfunktionellen aliphatischen Polyols hergestellt wird, dessen Esterfunktionen von einer gesättigten oder ethylenischungesättigten aliphatischen Monocarbonsäure mit wenigstens 14 Kohlestoffatomen stammen,

EP 0 394 314 B2

b) Zugeben eines kationischen, lipophilen Arzneimittels zu dem so erhaltenen sauren, liposomhaltigen wässrigen Medium, und

c) Zugeben einer Base, deren Kationen nicht die Lipiddoppelschicht der Liposomen durchdringen können, damit Säureanionen in der externen wässrigen Phase ladungsneutralisiert werden, wodurch der Übergang des kationischen, lipophilen Arzneimittels in die interne wässrige Phase der Liposomen induziert wird.

2. Verfahren nach Anspruch 1,
bei dem die monofunktionelle Pyranosidylsäure Glucarsäure, Gluconsäure, Gulonsäure, Galacturonsäure, Glucoheptonsäure oder Lactobionsäure ist.

3. Verfahren nach einem der vorhergehenden Ansprüche,
wobei die Säure Lactobionsäure ist.

4. Verfahren nach einem der vorhergehenden Ansprüche,
bei dem die Base ein Alkali- oder Erdalkalimetall-Hydroxid oder -Carbonat ist.

5. Verfahren nach einem der vorhergehenden Ansprüche,
bei dem nach der Zugabe der Base die Säure und andere Anionen aus der wässrigen Umgebung ausserhalb der Liposomen entfernt werden und durch eine wässrige Zuckerlösung ersetzt werden.

6. Verfahren nach einem der vorhergehenden Ansprüche,
bei dem das kationische, lipophile Arzneimittel ein anti-neoplastisches Anthracyclinmittel ist.

7. Verfahren nach Anspruch 1,
bei dem die Säure Zitronensäure, die Base Natriumhydroxid, der Diester Distearoyl-Phosphatidylcholin, das kationische, lipophile Arzneimittel Daunorubicin ist und in den Liposom-Doppelschichten Cholesterin als Stabilisator vorhanden ist.

8. Verfahren nach Anspruch 1,
bei dem die Säure Lactobionsäure, die Base Natriumhydroxid, der Diester Distearoyl-Phosphatidylcholin, das kationische, lipophile Mittel Doxorubicin ist und in den Liposom-Doppelschichten Cholesterin als Stabilisator vorhanden ist.

9. In Phospholipid eingeschlossene, kationische, lipophile Arzneimittelzusammensetzung, hergestellt nach einem der in den vorhergehenden Ansprüchen beschriebenen Verfahren.

**Revendications**

1. Procédé de préparation d'une composition médicamenteuse lipophile cationique emprisonnée dans un phospholipide comprenant les étapes consistant à :

a. former des liposomes dans un milieu aqueux contenant un acide qui a au moins un groupe monofonctionnel acide pyranosidylique ou acide citrique et où le pH du milieu aqueux est de 2,0 à 2,5 pour donner un milieu aqueux acide contenant des liposomes dans lequel l'acide est présent dans les phases liposomiques interne et externe, ledit liposome étant préparé à partir de diesters d'acide phosphatidyl-carboxylique à substitution hydroxyamino-aliphatique (inférieur) d'un polyol aliphatique à trois fonctions ou plus dans lequel les parties ester dérivent d'un acide monocarboxylique aliphatique saturé ou à insaturation éthylénique ayant au moins 14 atomes de carbone,

b. ajouter au milieu aqueux acide contenant des liposomes ainsi obtenu un médicament lipophile cationique, et

c. ajouter une base dont les cations ne peuvent pas passer à travers les bicouches lipidiques des liposomes pour neutraliser la charge des anions acides dans la phase aqueuse externe, induisant ainsi le passage du médicament lipophile cationique dans la phase aqueuse interne des liposomes.

2. Procédé selon la revendication 1 dans lequel l'acide pyranosidylique monofonctionnel est l'acide glucuronique, l'acide gluconique, l'acide gulonique, l'acide galacturonique, l'acide glucoheptanoïque ou l'acide lactobionique.

3. Procédé tel que décrit dans l'une quelconque des revendications précédentes dans lequel ledit acide est l'acide lactobionique.

4. Procédé tel que décrit dans l'une quelconque des revendications précédentes dans lequel ladite base est un hydroxyde ou un carbonate d'un métal alcalin ou alcalino-terreux.

5. Procédé tel que décrit dans l'une quelconque des revendications précédentes dans lequel, après addition de la base, l'acide et les autres anions sont enlevés de l'environnement aqueux externe aux liposomes et remplacés par une solution de sucre aqueuse.

6. Procédé tel que décrit dans l'une quelconque des revendications précédentes dans lequel ledit médicament lipophile cationique est un agent anti-néoplasique de type anthracycline.

7. Procédé tel que décrit dans la revendication 1 dans lequel ledit acide est l'acide citrique, ladite base est l'hydroxyde de sodium, ledit diester est la distéaroyl-phosphatidylcholine, ledit médicament lipophile cationique est la daunorubicine, et du cholestérol est présent dans les bicouches des liposomes comme stabilisant.

8. Procédé tel que décrit à la revendication 1 dans lequel ledit acide est l'acide lactobionique, ladite base est l'hydroxyde de sodium, ledit diester est la distéroyl-phosphatidylcholine, ledit médicament lipophile cationique est la doxorubicine et du cholestérol est présent dans les bicouches des liposomes comme stabilisant.

9. Composition médicamenteuse lipophile cationique emprisonnée dans un phospholipide préparée par un procédé tel que décrit dans l'une quelconque des revendications précédentes.